(19) **Europäisches Patentamt**

**European Patent Office**

**Office européen des brevets**

(11) Veröffentlichungsnummer: **0 269 848 B1**

(12) **EUROPÄISCHE PATENTSCHRIFT**

(45) Veröffentlichungstag der Patentschrift: **07.10.92**

(21) Anmeldenummer: **87115683.2**

(22) Anmeldetag: **26.10.87**

(51) Int. Cl.⁵: **A61N 1/08**

(54) **Reizstromgerät.**

(30) Priorität: **07.11.86 DE 3638067**

(43) Veröffentlichungstag der Anmeldung:
**08.06.88 Patentblatt 88/23**

(45) Bekanntmachung des Hinweises auf die
Patenterteilung:
**07.10.92 Patentblatt 92/41**

(84) Benannte Vertragsstaaten:
**AT BE CH DE FR LI NL**

(56) Entgegenhaltungen:
**US-A- 4 088 141**

**Schaltplan "UHER 4200 Report Monitor / 4400
Report Monitor"**

**Bedienungsanleitung "UHER 4200 Report
Monitor / 4400 Report Monitor", Seiten 8 und
9**

(73) Patentinhaber: **SIEMENS AKTIENGESELL-
SCHAFT
Wittelsbacherplatz 2
W-8000 München 2(DE)**

(72) Erfinder: **Helmreich, Klaus
Rhönstrasse 36
W-8520 Erlangen(DE)**
Erfinder: **Herzog, Ludwig
Drei-Thorn-Strasse 3
W-6948 Waldmichelbach(DE)**
Erfinder: **Knapp, Volker
Pestalozzistrasse 19
W-6948 Waldmichelbach(DE)**

# Beschreibung

Die Erfindung bezieht sich auf ein Reizstromgerät gemäß Oberbegriff des Patentanspruchs 1.

Aus dem Schaltplan "UHER 4200 Report Monitor/4400 Report Monitor" ist ein Mikrofon-Radio-Verstärker bekannt, bei dem ein erstes Potentiometer in einer speziellen Art zu einem zweiten Potentiometer parallel geschaltet ist. Diese Parallelschaltung wird erreicht, indem an dem ersten Potentiometer der Schleifer und ein Anschluß des Festwiderstandes mit je einem Anschluß des Festwiderstandes an dem zweiten Potentiometer verbunden sind. Der Schleifer an dem zweiten Potentiometer ist mit dem Eingang einer Verstärkerstufe verbunden. In der bekannten Schaltung wird das Signal über das erste und zweite Potentiometer (= Einstellglieder) zu dem Verstärker geführt. Gemäß der Bedienungsanleitung "UHER 4200 Report Monitor/4400 Report Monitor", Seiten 8 und 9, wird mit dem ersten Potentiometer die Aussteuerung eingestellt. Das zweite Potentiometer dient gemäß Bedienungsanleitung zum synchronen Ein- und Ausblenden eines Stereosignals oder des Summensignals bei Mono-Aufnahmen, wenn zwei Signalquellen gemischt werden. Zur Einstellung der Aussteuerung muß gemäß Bedienungsanleitung zuvor das zweite Potentiometer aber mindestens bis zur Ziffer 8 einer 10 Ziffern umfassenden Skala aufgedreht werden.

Aus der JP-A 56-122238 in Patent Abstracts of Japan, Band 5, Nr. 198, (E-87) (870), 16. Dezember 1981, ist es bei einem Frequenzgenerator zur Verstärkung eines Ausgangssignales in Abhängigkeit von einer Frequenz bekannt, eine von einem Frequenzeinstellglied gelieferte Spannung mittels eines A/D-Wandlers digital in einem Speicher zu speichern und mit dem jeweils gespeicherten Wert die Ausgangsspannung eines Verstärkers zu steuern.

Aus der US-A-4,088,141 ist ein Nervenstimulator bekannt, der automatisch immer dann abgeschaltet wird, wenn an einem Intensitätseinstellglied ein zu hoher Ausgangsstrom eingestellt wird, der einen maximal zulässigen Stromwert überschreitet.

Intensitätseinstellglieder sind bekanntlich Toleranzschwankungen unterworfen. Dasselbe gilt z.B. auch für nachgeschaltete Analog-Digital-Wandler bei digitaler Verarbeitung. Der tatsächliche intensitätseinstellbereich sollte aber immer derselbe sein, d.h. bei Nullstellung des Intensitätseinstellgliedes sollte die Intensität tatsächlich Null (0 mA) bei Höchstwerteinstellung der Höchstwert (z.B. 70 mA) sein.

Aufgabe vorliegender Erfindung ist es, ein Reizstromgerät aufzubauen, das in diesem Sinne besonders einfach zu eichen ist.

Die Aufgabe wird erfindungsgemäß durch die Merkmale des Patentanspruchs 1 gelöst.

Eine vorteilhafte Ausgestaltung der Erfindung mit Mikroprozessor ergibt sich aus Anspruch 7. Besonders vorteilhaft für Reizstromgeräte ist auch die Ausgestaltung gemäß Anspruch 8. Hier lassen sich quasilogarithmische Kennlinienverläufe erzeugen.

Weitere Vorteile und Einzelheiten der Erfindung ergeben sich aus der nachfolgenden Beschreibung eines Ausführungsbeispieles anhand der Zeichnung und in Verbindung mit den weiteren Unteransprüchen.

Fig. 1      das erfindungsgemäße Reizstromgerät im Prinzipschaltbild,

Fig. 2      eine Draufsicht auf einen Drehknopf eines Drehpotentiometers als Bedienungselement für das Intensitätseinstellglied und

Fig. 3 bis 5      Diagramme, die den Anpaßmechanismus wiedergeben.

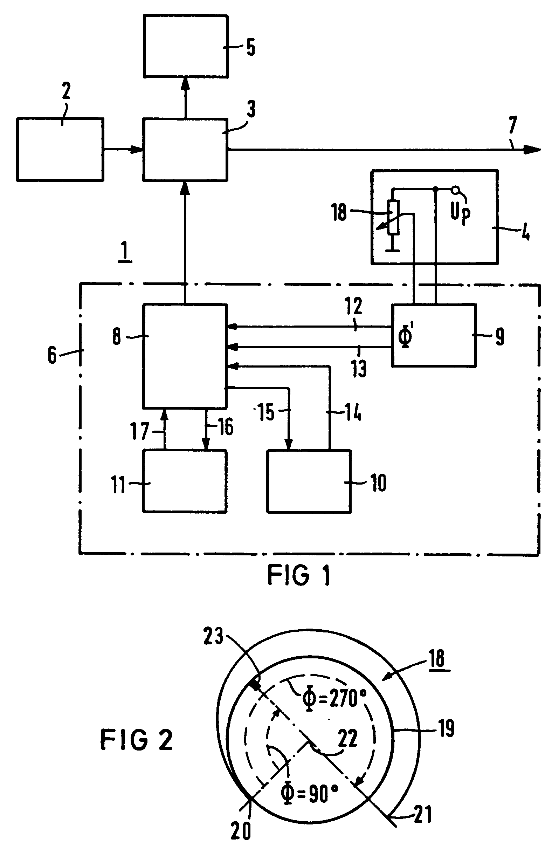

In der Fig. 1 umfaßt das Reizstromgerät 1 unter anderem einen Reizsignalerzeuger 2, eine Intensitätseinstelleinrichtung 3 mit Intensitätseinstellgleid 4 und Intensitätseinstellanzeige 5 sowie eine Intensitätsbereichsanpassungsvorrichtung 6. Die am Ausgang der Intensitätseinstelleinrichtung 3 anfallenden Reizsignale werden in Pfeilrichtung 7 zum Reizausgang des Reizstromgerätes mit den angeschlossenen Reizstromelektroden (nicht dargestellt) weitergeleitet.

Die Intensitätsbereichsanpassungsvorrichtung 6 umfaßt einen Mikroprozessor 8, einen Analog-Digital-Wandler 9, ein RAM 10 und ein EPROM 11. Die drei zuletzt genannten Bauelemente sind über Leitungen 12 bis 17 mit dem Mikroprozessor 8 verbunden.

Das Intensitätseinstellglied 4 umfaßt ein Drehpotentiometer 18, das an der Spannung $U_P$ liegt.

Gemäß Fig. 2 kann das Drehpotentiometer 18 mittels Drehknopf 19 um $\phi = 270°$ aus einer Nullstellung 20 in die Höchstwertstellung 21 um die Drehachse 22 gedreht werden. Der Positionsanzeiger des Drehknopfes ist mit 23 angedeutet.

Das Drehpotentiometer 18 ist so gestaltet, daß es quasilogarithmisch von etwa $\phi = 0°$ bis 90° eine nur sehr langsam von $\phi = 90°$ bis 270° hingegen eine relativ steil linear ansteigende Ausgangsspannung liefert. Die Kennlinie weist also einen Knick bei ca. 90° auf. Die Einstellcharakteristik ist also zur feinfühligen Dosierung bei kleinen Strömen gespreizt.

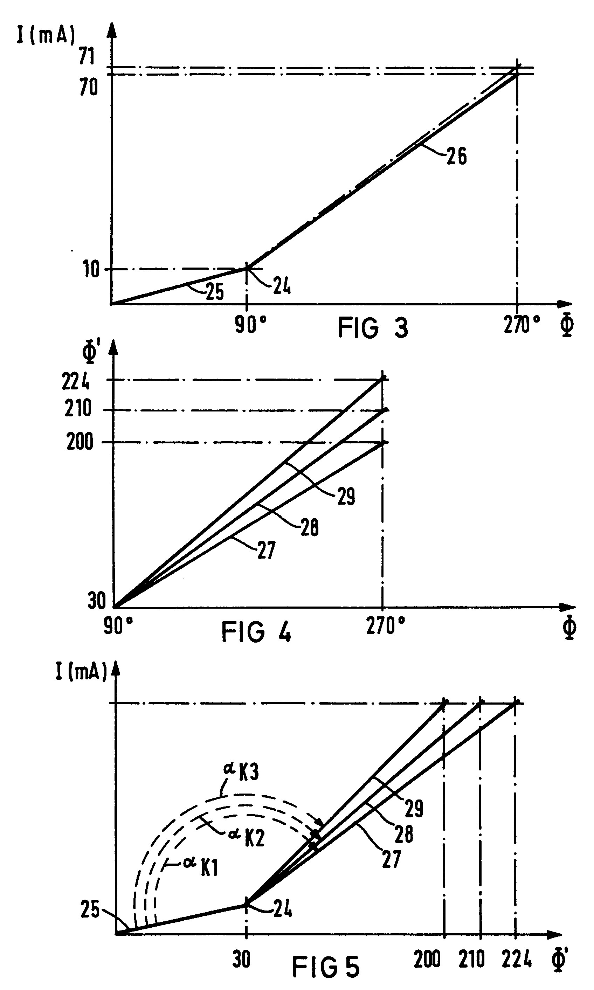

Wünschenswert wäre ein Verlauf der Kennlinie, wie er z.B. in Fig. 3 dargestellt ist. Der Knickpunkt 24 bei $\phi = 90°$ und der Höchstwert bei $\phi = 270°$ liegen so, daß der Intensitätsbereich zwischen z.B. 10 mA bis 70 mA exakt eingehalten ist.

Leider ergeben sich nun aber auf Grund von Toleranzschwankungen Abweichungen des Höchstwertes. In der Fig. 3 ist z.B. bei $\phi$ = 270° der Höchstwert um 1 mA auf 71 mA erhöht.

Die Intensitätsbereichsanpassungsvorrichtung 6 unternimmt nun eine Bereichsanpassung wie folgt: Das Kennlinienteilstück 25 von $\phi$ = 0° bis 90° unterhalb des Knickpunktes 24 ist von Toleranzschwankungen relativ wenig beeinflußt. Es wird fest im EPROM 11 eingespeichert. Das Kennlinienteilstück 26 wird hingegen entsprechend den Fig. 4 und 5 angepaßt.

Fig. 4 zeigt das Ausgangssignal $\phi'$ (digitale Zahl) des Analog-Digital-Wandlers 9 bei unterschiedlichen Drehwinkeln $\phi$ des Drehpotentiometers 18 und drei unterschiedlichen Toleranzbereichen. Bei $\phi$ = 90° ist z.B. $\phi'$ = 30 für alle Toleranzbereiche. Bei $\phi$ = 270° ergibt sich für drei verschiedene Toleranzbereiche $\phi'$ = 200, 210, 224.

Der Mikroprozessor 8 erfaßt nun den jeweiligen Höchstwert $\phi'$ = 200, 210 oder 214 und berechnet danach ausgehend vom Ausgangswert $\phi'$ = 30 ein Kennlinienteilstück 27, 28 oder 29, dessen Knickwinkel $\alpha_{K1}$, $\alpha_{K2}$ oder $\alpha_{K3}$ im Knickpunkt 24 so ist, daß es mit $\phi'$ = 200, 210 oder 224 auf der Abszisse immer dieselbe Höchstwertkoordinate bei 70 mA aufweist. Dieses Kennlinienteilstück 27, 28 oder 29 wird nun vom Mikroprozessor 8 im RAM 10 abgespeichert. Zusätzlich kopiert der Mikroprozessor 8 auch noch das im EPROM 11 fest eingespeicherte Kennlinienteilstück 25 in das RAM 10, wo es in Anfügung an das Kennlinienteilstück 27, 28 oder 29 zum Gesamtkennlinienverlauf zusammengesetzt wird. Dieser Vorgang ist in Fig. 5 mit dem Diagramm I($\phi'$) verdeutlicht.

Der Intensitätsbereich der Intensitätseinstellvorrichtung 3 ist jetzt also unabhängig von Toleranzschwankungen des Intensitätseinstellgliedes 4. Jeder vom Intensitätseinstellglied 4 gelieferte Intensitätseinstellwert wird also mit Hilfe des Mikroprozessors 8 und des RAM 10 in den richtigen zum Einstellbereich passenden Wert umgewandelt.

Die Eichung selbst kann einmalig vor oder mehrfach (z.B. periodisch) während einer Reizstromtherapie erfolgen.

**Patentansprüche**

1. Reizstromgerät, insbesondere für Reizstromtherapie an einem Patienten, mit einem Reizsignalerzeuger (2), dessen Reizsignal über eine Intensitätseinstelleinrichtung (3) zu den am Reizausgang angeschlossenen Reizstromelektroden geleitet wird, wobei die Intensitätseinstelleinrichtung (3) mit einem Intensitätseinstellglied (4) verbunden ist, das einem Höchstwert für die Intensitätseinstellung umfaßt, **dadurch gekennzeichnet,** daß zwischen Intensitätseinstelleinrichtung (3) und Intensitätseinstellglied (4) eine Intensitätsbereichsanpassungsvorrichtung (6) eingeschaltet ist, die zur Anpassung des Istbereiches des Intensitätseinstellgliedes (4) in Abhängigkeit wenigstens von dessen Höchstwert an einen vorgebbaren Intensitätsbereich der Intensitätseinstelleinrichtung (3) einen Mikroprozessor (8) umfaßt.

2. Reizstromgerät nach Anspruch 1, **dadurch gekennzeichnet,** daß die Intensitätsbereichsanpassungsvorrichtung (6) im Sinne einer Linearanpassung wirkt.

3. Reizstromgerät nach Anspruch 1 oder 2, **dadurch gekennzeichnet,** daß der Anfangswert der Anpassung zu einem Wert im etwa ersten Drittel der Intensitätseinstellung am Intensitätseinstellglied (4) gewählt ist.

4. Reizstromgerät nach Anspruch 3, **dadurch gekennzeichnet,** daß das Intensitätseinstellglied (4) ein Drehpotentiometer (18) umfaßt, das zwischen etwa $\phi$ = 0° und 270° drehbar ist und daß der Anfangswert (24) der Anpassung bei einem Intensitätswert liegt, der einem Drehwinkel des Drehpotentiometers von etwa $\phi$ = 90° entspricht.

5. Reizstromgerät nach Anspruch 3 oder 4, **dadurch gekennzeichnet,** daß der Intensitätsbereich zwischen Null und dem Anfangswert der Anpassung unabhängig vom Höchstwert der Einstellung des Intensitätseinstellgliedes (4) vorgegeben ist.

6. Reizstromgerät nach Anspruch 5, **dadurch gekennzeichnet,** daß der Intensitätsbereich zwischen Null und dem Anfangswert von einem Festspeicher (11), insbesondere EPROM, abrufbar ist.

7. Reizstromgerät nach einem der Ansprüche 1 bis 6, **dadurch gekennzeichnet,** daß die Intensitätsbereichsanpassungsvorrichtung (6) einen weiteren Speicher (10) und einen Analog-Digital-Wandler (9) umfaßt, wobei der Analog-Digital-Wandler wenigstens den Höchstwert der Einstellung am Intensitätseinstellglied (4) erfaßt, der dann vom Mikroprozessor (8) abgerufen und zusammen mit dem Anfangswert der Anpassung zur Berechnung einer stückweisen Intensitätsbereichskennlinie (27, 28 oder 29) im vorgegebenen Intensitätsbereich zwischen Anfangswert und Höchstwert herangezogen wird, welche Intensitätsbereichskennlinie im weiteren Speicher (10) abgelegt und zur Anpassung tatsächlich eingestellter Istwerte am Intensitätseinstellglied (4) durch den Mikroprozessor (8) an der Intensitätseinstellrichtung (3) herangezogen wird.

8. Reizstromgerät nach Anspruch 6 und 7, **dadurch gekennzeichnet,** daß das im Festspeicher (11) gespeicherte Kennlinienteilstück (25) zwischen Null und dem Anfangswert der Anpassung vom Mikroprozessor (8) abrufbar und in den weiteren Speicher (10) zur Zusammensetzung zusammen mit der stückweisen Intensitätsbereichskennlinie zur gesamten Intensitätsbereichskennlinie von Null bis Höchstwert einspeicherbar ist.

9. Reizstromgerät nach Anspruch 8, **dadurch gekennzeichnet,** daß das Kennlinienstück (25) zwischen Null und dem Anfangswert und das Kennlinienstück (27, 28 oder 29) zwischen dem Anfangswert und dem Höchstwert linear sind und unterschiedliche Steigungen aufweisen.

10. Reizstromgerät nach Anspruch 9, **dadurch gekennzeichnet,** daß das lineare Kennlinienstück zwischen Anfangswert und Höchstwert steiler ist als jenes zwischen Null und Anfangswert.

11. Reizstromgerät nach einem der Ansprüche 7 bis 10, **dadurch gekennzeichnet,** daß der weitere Speicher (10) ein RAM ist.

**Claims**

1. A stimulus current apparatus in particular for stimulus current therapy on a patient, with a stimulus signal generator (2), the stimulus signal of which is led by way of an intensity adjusting device (3) to the stimulus current electrodes connected to the stimulus output, wherein the intensity adjusting device (3) is connected to an intensity adjusting element (4), which includes a maximum value for the intensity adjustment, characterised in that between the intensity adjusting device (3) and the intensity adjusting element (4) there is connected an intensity range adaption device (6), which comprises a microprocessor (8) for adapting the actual range of the intensity adjusting element (4) in dependence on at least the maximum value thereof to a preset intensity range of the intensity adjusting device (3).

2. A stimulus current apparatus according to claim 1, characterised in that the intensity range adaption device (6) acts for the purpose of a linear adaption.

3. A stimulus current apparatus according to claim 1 or 2, characterised in that the initial value of the adaption to one value is selected in approximately the first third of the intensity adjustment at the intensity adjusting element (4).

4. A stimulus current apparatus according to claim 3, characterised in that the intensity adjusting element (4) comprises a rotary potentiometer (18), which is able to be rotated between approximately $\phi = 0°$ and $270°$ and in that the initial value (24) of the adaption lies at an intensity value which corresponds to an angle of rotation of the rotary potentiometer of approximately $\phi = 90°$.

5. A stimulus current apparatus according to claim 3 or 4, characterised in that the intensity range between nil and the initial value of the adaption is specified independently of the maximum value of the adjustment of the intensity adjusting element (4).

6. A stimulus current apparatus according to claim 5, characterised in that the intensity range between nil and the initial value is able to be called up by a read-only memory (11), in particular EPROM.

7. A stimulus current apparatus according to one of claims 1 to 6, characterised in that the intensity range adaption device (6) includes a further memory (10) and an analog-digital converter (9), wherein the analog-digital converter acquires at least the maximum value of the adjustment at the intensity adjusting element (4), which can then be called up by the microprocessor (8) and together with the initial value of the adaption is brought up for calculating a piecewise intensity range characteristic curve (27, 28 or 29) in the specified intensity range between the initial value and the maximum value, which intensity range characteristic curve is deposited in the further memory (10) and is brought up at the intensity adjusting device (3) by the microprocessor (8) for adapting the actually adjusted actual values at the intensity adjusting element (4).

8. A stimulus current apparatus according to claim 6 and 7, characterised in that the partial piece of the characteristic curve (25) stored in the read-only memory (11) between nil and the initial value of the adaption is able to be called up by the microprocessor (8) and stored in the further memory (10) for combining together with the piecewise intensity range characteristic curve to (form) the total intensity range characteristic curve from nil to the maximum value.

9. A stimulus current apparatus according to claim 8, characterised in that the part of the characteristic curve (25) between nil and the initial value and the part of the characteristic curve (27, 28 or 29) between the initial value and the maximum value are linear and have varying gradients.

10. A stimulus current apparatus according to claim 9, characterised in that the linear part of the characteristic curve between the initial value and the maximum value is steeper than that between nil and the initial value.

11. A stimulus current apparatus according to one of claims 7 to 10, characterised in that the further memory (10) is a RAM.

**Revendications**

1. Stimulateur, notamment pour la mise en oeuvre d'une thérapie de stimulation chez un patient, comportant un générateur de signaux de stimulation (2), dont le signal de stimulation est envoyé par l'intermédiaire d'un dispositif de réglage (3) de l'intensité aux électrodes de stimulation raccordées à la sortie du signal de stimulation, le dispositif (3) de réglage de l'intensité étant raccordé à un circuit (4) de réglage de l'intensité, qui comporte une valeur maximale pour le réglage de l'intensité, caractérisé par le fait qu'entre le dispositif (3) de réglage de l'intensité et le circuit (4) de réglage de l'intensité est branché un dispositif (6) d'adaptation de la plage des intensités, qui comprend un microprocesseur (8) pour l'adaptation de la plage des valeurs réelles contenues dans le circuit (4) de réglage de l'intensité, en fonction au moins de la valeur maximale contenue dans ce circuit, à une plage, pouvant être prédéterminée, d'intensités du dispositif (3) de réglage de l'intensité.

2. Stimulateur suivant la revendication 1, caractérisé par le fait que le dispositif (6) d'adaptation de la plage des intensités agit dans le sens d'une adaptation linéaire.

3. Stimulateur suivant la revendication 1 ou 2, caractérisé par le fait que la valeur initiale de l'adaptation à une valeur est choisie approximativement dans le premier tiers du réglage de l'intensité dans le circuit (4) de réglage de l'intensité.

4. Stimulateur suivant la revendication 3, caractérisé par le fait que le circuit (4) de réglage de l'intensité comprend un potentiomètre rotatif (18), qui peut tourner entre environ $\phi = 0°$ et $270°$ et que la valeur initial (24) de l'adaptation se situe à une valeur de l'intensité qui correspond à un angle de rotation du potentiomètre rotatif égal à environ $\phi = 90°$.

5. Stimulateur suivant la revendication 3 ou 4, caractérisé par le fait que la plage des intensités est prédéterminée entre la valeur zéro et la valeur initiale de l'adaptation, indépendamment de la valeur maximale du réglage du circuit (4) de réglage de l'intensité.

6. Stimulateur suivant la revendication 5, caractérisé par le fait que la plage des intensités entre la valeur zéro et la valeur initiale peut être appelée à partir d'une mémoire morte (11), notamment une mémoire EPROM.

7. Stimulateur suivant l'une des revendications 1 à 6, caractérisé par le fait que le dispositif (6) d'adaptation de la plage des intensités comporte une autre mémoire (10) et un convertisseur analogique/numérique (9), le convertisseur analogique/numérique détectant au moins la valeur maximale du réglage dans le circuit (4) de réglage de l'intensité, qui est appelé par le microprocesseur (8) et est utilisé, conjointement avec la valeur initiale de l'adaptation, pour le calcul d'une courbe caractéristique de la plage des intensités (27,28 ou 29), formée d'éléments, dans la plage des intensités prédéterminée entre la valeur initiale et la valeur maximale, laquelle courbe caractéristique de la plage des intensités est mémorisée dans l'autre mémoire (10) et est utilisée pour réaliser l'adaptation de valeurs réelles effectivement réglées dans le circuit (4) de réglage de l'intensité, par le microprocesseur (8), au dispositif (3) de réglage de l'intensité.

8. Stimulateur suivant l'une des revendications 6 et 7, caractérisé par le fait que l'élément de courbe caractéristique (25) mémorisé dans la mémoire morte (11) peut être appelé entre la valeur zéro et la valeur initiale de l'adaptation par le microprocesseur (8) et peut être mémorisé dans l'autre mémoire (10) pour être réuni à la courbe caractéristique de la plage des intensités, formées d'éléments, pour constituer l'ensemble de la courbe caractéristique de plages des intensités de zéro jusqu'à la valeur maximale.

9. Stimulateur suivant la revendication 8, caractérisé par le fait que l'élément (25) de la courbe caractéristique entre zéro et la valeur initiale et l'élément (27,28 ou 29) de la courbe caractéristique entre la valeur initiale et la valeur maximale sont linéaires et possèdent des pentes différentes.

10. Stimulateur suivant la revendication 9, caractérisé par le fait que l'élément linéaire de la courbe caractéristique entre la valeur initiale et la valeur maximale est plus pentu que chaque élément compris entre zéro et la valeur initiale.

11. Stimulateur suivant l'une des revendications 7 à 10, caractérisé par le fait que l'autre mémoire (10) est une mémoire RAM.

FIG 1

FIG 2

FIG 3

FIG 4

FIG 5